# EUROPEAN PATENT APPLICATION

(11) **EP 4 574 104 A1**
(43) Date of publication of application: **25.06.2025**
(21) Application number: 24220581.3
(22) Date of filing: 17.12.2024
(51) Int. Cl.: A61F 5/44

(54) **URINARY BAG REDUCING URINE BLOCKAGE**

(30) Priority: 19.12.2023 TW 112149599
(71) Applicant: Archer Biotechnology Development Co., Ltd., Taichung City 404034 (TW); Su, Chien-Chung, Taichung City 404034 (TW); Chang, Yi-Chia, Taichung City 404034 (TW); Ku, Ya-Wen, Taichung City 404034 (TW)
(72) Inventor: SU, Chien-Chung, 404034 Taichung City (TW); CHANG, Yi-Chia, 404034 Taichung City (TW); KU, Ya-Wen, 404034 Taichung City (TW); HSU, Ping-Yi, 404034 Taichung City (TW)
(74) Representative: Viering, Jentschura & Partner mbB Patent- und Rechtsanwälte

(57) **Abstract**

An urinary bag reducing urine blockage includes an urinary bag body (10) having a through bore (12), a catheter (20), and a bend preventing tube (30). The catheter (20) has one end connected with an urethra of a user, and the other end passing through the through bore (12) of the urinary bag body (10). The bend preventing tube (30) has a flow bore (31) therein, and includes an insertion part (32) and a supporting part (33). The catheter (20) is in communication with the flow bore (31) and mounted around the insertion part (32). The insertion part (32). keeps the catheter (20) to be straight. The supporting part (33) is located inside the urinary bag body (10). The outer diameter of the supporting part (33) is larger than the outer diameter of the insertion part (32). The supporting part (33) supports the interior of the urinary bag body (10). Thus, the bend preventing tube (30) prevents the catheter (20) and the urinary bag body (10) from bending to cause urine blockage, ensuring smooth urine drainage.

## Description

### BACKGROUND OF THE INVENTION

### 1. Field of the Invention:

The present invention relates to urinary bag structures, and more particularly, to an urinary bag reducing urine blockage.

### 2. Description of the Related Art:

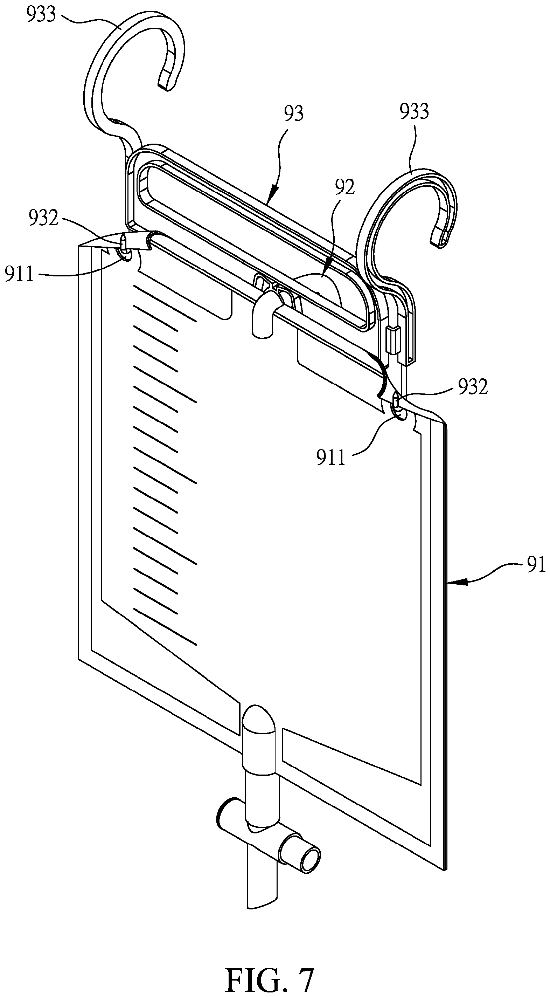

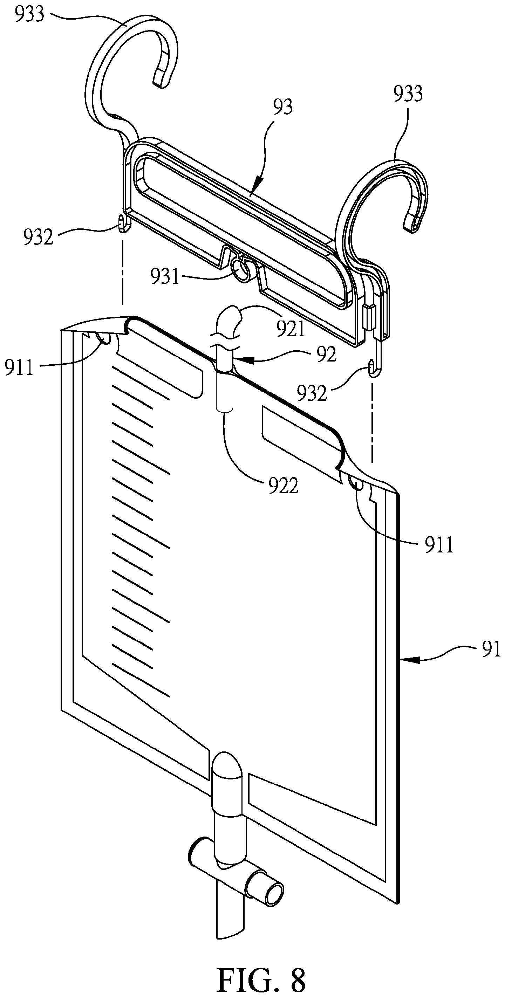

Patients are unable to urinate voluntarily (such as those undergoing or underwent surgery) or individuals with disabilities usually require the use of an urinary bag. Referring to **Fig. 7** and **Fig. 8****,** a conventional urinary bag structure comprises an urinary bag body **91,** a catheter **92,** and a hanger **93.** The two upper corners of the urinary bag body **91** are provided with two fixing bores **911.** The catheter **92** has a first connection part **921** and a second connection part **922.** The first connection part **921** is connected to the urethra of the user, while the second connection part **922** passes through the urinary bag body **91** for facilitating the communication therewith. A through hole **931** is provided at the lower middle position of the hanger **93,** with two hooking parts **932** extending downward and two hooks **933** extending upward on both sides of the hanger **93.**

During use, the catheter **92** passes through the through hole **931** of the hanger **93,** and the two hooking parts **932** of the hanger **93** are hooked onto the two fixing bores **911** of the urinary bag body **91.** The two hooks **933** of the hanger **93** are hooked onto the bedside or other suitable locations, thereby allowing the urine of the user to flow into the urinary bag body **91** via the catheter **92** for urine collection.

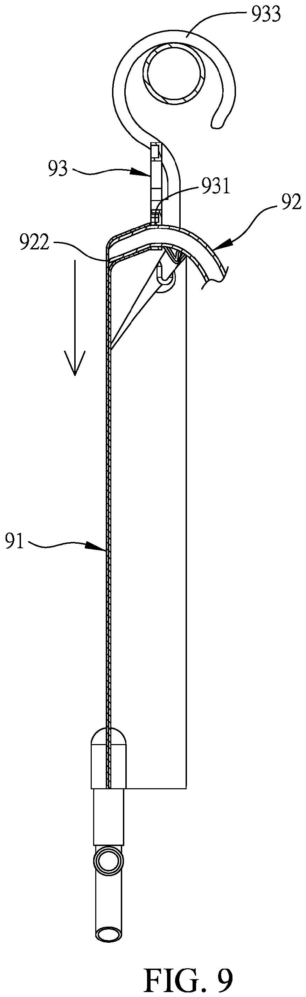

However, in conventional urinary bag structures, the urinary bag body **91** often deforms due to the weight of the collected urine, as shown in **Fig. 9****.** This deformation causes the catheter **92** to be bent in adjacent to the urinary bag body **91,** such that the second connection part **922** of the catheter **92** is pressed against the inner wall of the urinary bag body **91,** resulting in the urine blockage issue hindering the normal urinary drainage process. Also, due to the structural complexity, the assembly of the conventional urinary bag is inconvenient, and the manufacturing cost thereof is relatively high, failing to meet the economic efficiency.

### SUMMARY OF THE INVENTION

In view of the aforementioned issues of conventional urinary bag structures, the inventor of the present invention invented the idea through extensive discussions, sample testing, and multiple iterations of revisions and improvements, whereby the present invention is accordingly introduced.

An embodiment of the present invention provides an urinary bag reducing urine blockage, comprising:
an urinary bag body having a through bore on an upper side thereof, the through bore in communication with an interior of the urinary bag body;
a catheter connected and in communication with the urinary bag body, the catheter having a first connection part and an opposite second connection part, the first connection part used to be connected with an urethra of a user, the second connection part inserted into the urinary bag body from the through bore and placed inside the urinary bag body; and
a bend preventing tube connected and in communication between the urinary bag body and the catheter, the bend preventing tube having an insertion part and a supporting part, with a flow bore passing through the insertion part and the supporting part, the second connection part of the catheter mounted around the insertion part, the flow bore in communication with the catheter, the insertion part keeping the catheter to be straight; the supporting part located inside the urinary bag body, an outer diameter of the supporting part being larger than an outer diameter of the insertion part, the supporting part supporting the interior of the urinary bag body.

With such configuration, the urinary bag reducing urine blockage disclosed by the present invention comprises the bend preventing tube disposed between the catheter and the urinary bag body, wherein the insertion part of the bend preventing tube keeps the catheter to be straight, and the supporting part of the catheter supports the urinary bag body. Therefore, the urine blockage issues is prevented, ensuring the normal operation of urine drainage, thereby resolving the issues of the conventional urinary bag being easily bent and suffering from urine blockage.

Also, the present invention is structurally simple, facilitating a quick assembly of the urinary bag, and lowering the overall manufacturing cost, meeting the economic efficiency.

### BRIEF DESCRIPTION OF THE DRAWINGS

**Fig. 1** is a perspective view of the present invention.
**Fig. 2** is an exploded view of the present invention.
**Fig. 3** is a sectional view of the present invention.
**Fig. 4** is an enlarged view of part **A1** in **Fig. 3****.**
**Fig. 5** is a schematic view illustrating the operation status of the present invention.
**Fig. 6** is an enlarged view of part **A2** in **Fig. 5****.**
**Fig. 7** is a perspective view of a conventional urinary bag.
**Fig. 8** is a sectional view of a conventional urinary bag.
**Fig. 9** is a schematic view illustrating the operation status of a conventional urinary bag.

### DETAILED DESCRIPTION OF THE INVENTION

The aforementioned and further advantages and features of the present invention will be understood by reference to the description of the preferred embodiment in conjunction with the accompanying drawings where the components are illustrated based on a proportion for explanation but not subject to the actual component proportion.

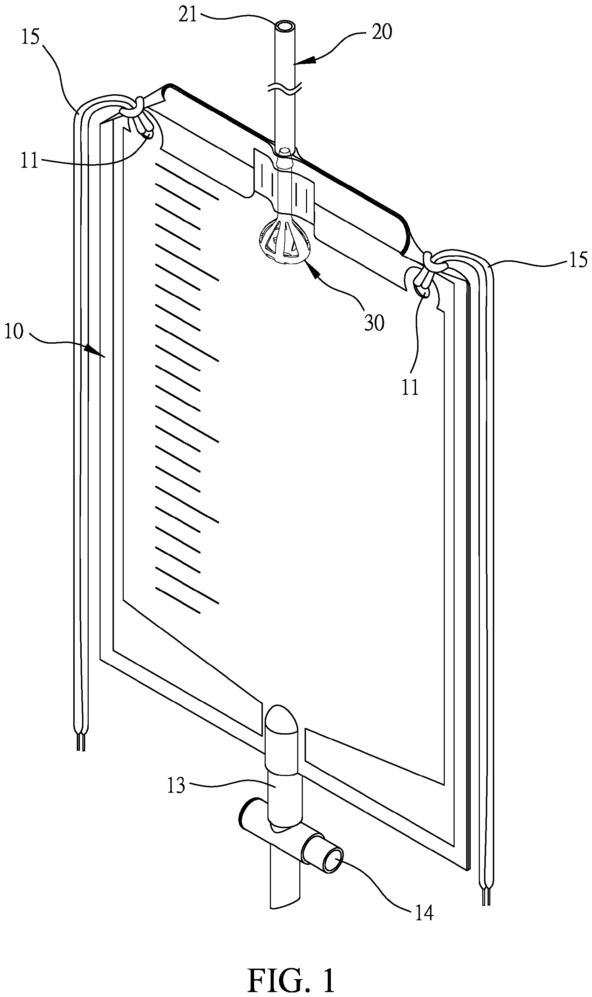

Referring to **Fig. 1** to **Fig. 4****,** the present invention provides an urinary bag reducing urine blockage comprises an urinary bag body **10,** a catheter **20,** and a bend preventing tube **30.**

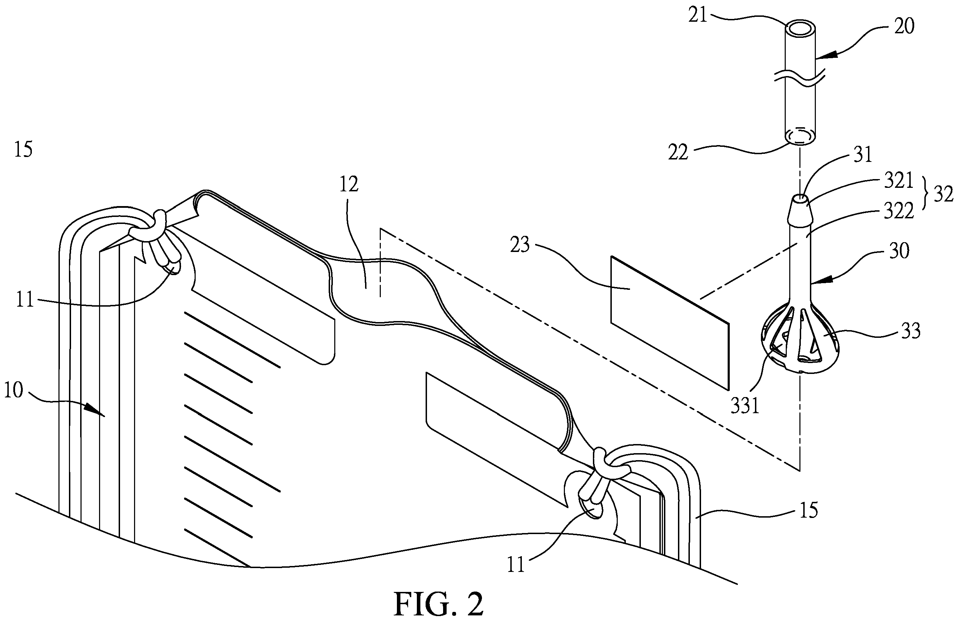

The urinary bag body **10** comprises two fixing bores **11** passing through two upper corners of the urinary bag body **10,** and a through bore **12** on an upper middle side of the urinary bag body **10.** The through bore **12** is arranged between the two fixing bores **11** (as shown in **Fig. 1** and **Fig. 2**). The through bore **12** is in communication with the interior of the urinary bag body **10,** and the urinary bag body **10** accommodates urine.

Also, an urine discharging tube **13** is connected to a lower middle side of the urinary bag body **10,** with a valve device **14** disposed on the urine discharging tube **13,** as shown in **Fig. 1****.** Two positioning bands **15** are disposed in the two fixing bores **11,** so that the urinary bag body **10** is allowed to be hung on the needed location by use of the two positioning bands **15.**

The catheter **20** comprises a first connection part **21** and an opposite second connection part **22.** The first connection part **21** is used to be connected with the urethra of the user (not shown), and the second connection part **22** is inserted into the urinary bag body **10** from the through bore **12** and placed inside the urinary bag body **10.** Therein, the through bore **12** of the urinary bag body **10** is sealed through a processing method, which tightly fixes the catheter **20** in the through bore **12** of the urinary bag body **10.** Therein, the processing method is allowed to be high-frequency welding or ultrasonic welding. In the embodiment, the processing method is high-frequency welding.

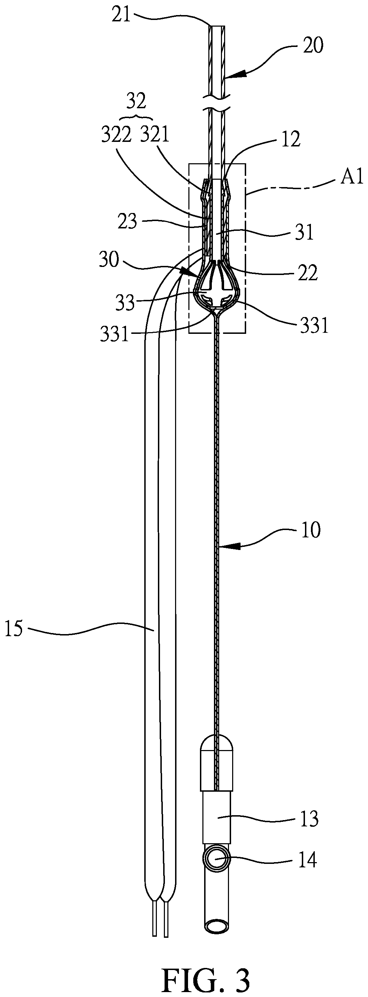

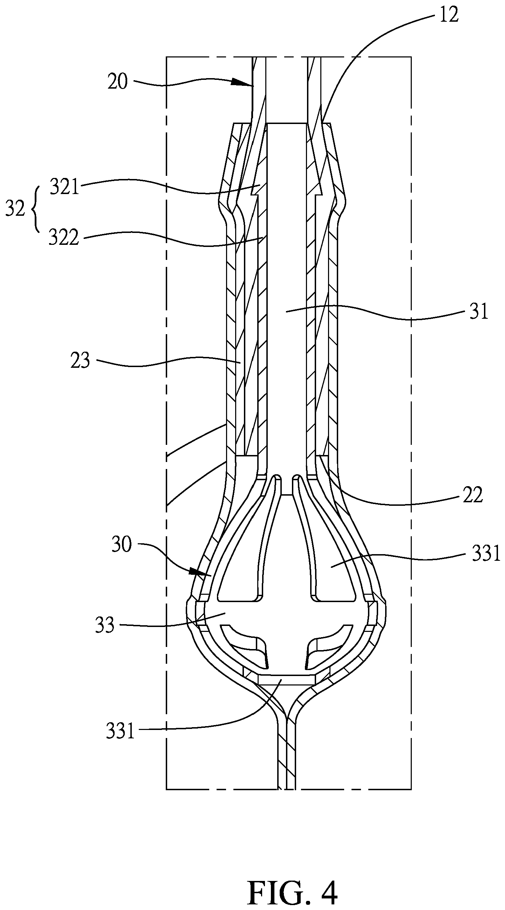

Also, a plastic sheet **23** is disposed between the second connection part **22** of the catheter **20** and the urinary bag body **10.** The plastic sheet **23** wraps between the second connection part **22** of the catheter **20** and the urinary bag body **10** (as shown in **Fig. 3** and **Fig. 4**). The plastic sheet **23** increases the tightness of the through bore **12** being sealed through the processing method, so as to achieve a tighter fixation of the urinary bag body **10** and the catheter **20,** preventing the urine from refluxing or leaking from the second connecting portion **22.**

The bend preventing tube **30** is connected between and in communication with the urinary bag body **10** and the catheter **20.** The bend preventing tube **30** is integrally formed of a rigid plastic material, with a flow bore **31** passing therethrough. The bend preventing tube **30** comprises an insertion part **32** and an opposite supporting part **33.** The second connection part **22** of the catheter **20** is mounted around the insertion part **32.** The flow bore **31** is in communication with the catheter **20.** The insertion part **32** keeps the catheter **20** to be straight. The supporting part **33** is placed inside the urinary bag body **10,** as shown in **Fig. 3** and **Fig. 4****.**

Also, the insertion part **32** comprises a first portion **321** and a second portion **322.** The first portion **321** is formed in a cone shape, and the second portion **322** is formed in a column shape. The largest outer diameter of the first portion **321** is larger than the outer diameter of the second portion **322;** the length of the second portion **322** is larger than the length of the first portion **321,** as shown in **Fig. 3** and **Fig. 4****.** Therein, when the catheter **20** is to be mounted around the insertion part **32** of the bend preventing tube **30,** the second connection part **22** of the catheter **20** is efficiently mounted around the first portion **321** which has the smallest outer diameter. Then, with the variation from the expanding outer diameter of the first portion **321** to the smaller outer diameter of the second portion **322,** the catheter **20** is stably mounted around the insertion part **32,** achieving a detachment preventing effect.

Also, the outer diameter of the supporting part **33** is larger than the outer diameter of the first portion **321** of the insertion part **32.** The supporting part **33** is able to support the interior of the urinary bag body **10.** In the embodiment, the outer diameter of the supporting part **33** is larger than the largest diameter of the insertion part **32.**

In addition, the supporting part **33** is formed in an ellipsoidal shape. A plurality of hollow holes **331** are distributed on the circumferential surface of the supporting part **33,** and each hollow hole **331** is in communication with the flow bore **31.** Therein, each hollow hole **331** is formed in a triangular shape, polygonal shape, or a combination thereof. In the embodiment, each hollow hole **331** is formed in the combination of triangular shape and polygonal shape. However, in the present invention, each hollow hole **331** is also allowed to be formed in other geometric shapes, not limited thereto.

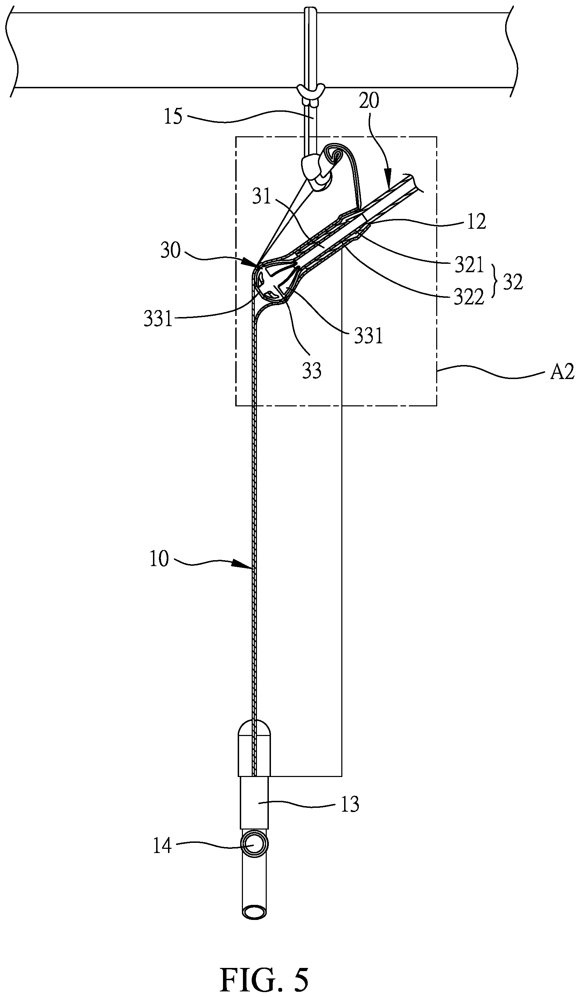

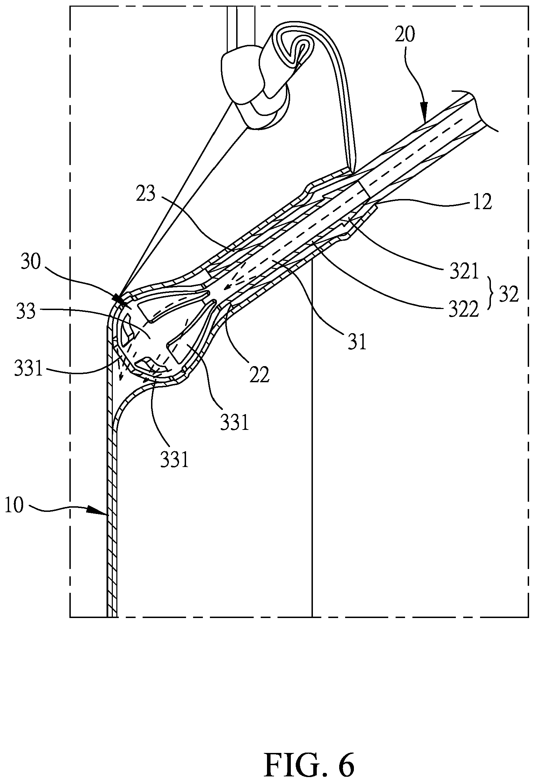

Referring to **Fig. 5** and **Fig. 6****,** during use, the two positioning bands **15** are fastened to the bedside or other suitable locations, and the urine of the user flows into the urinary bag body **10** through the catheter **20** to be collected. At the position where the catheter **20** normally bends in adjacent to the urinary bag body **10,** the insertion part **32** keeps the catheter **20** to be straight, preventing the catheter **20** from being affected by the urinary bag body **10** to be bent. Furthermore, the supporting part **33** of the bend preventing tube **30** is pressed against the inner wall of the urinary bag body **10,** so as to support the interior of the urinary bag body **10.** Therefore, the urine flows from the catheter **20** through the flow bore **31** of the bend preventing tube **30** into the supporting part **33,** and then smoothly enters the urinary bag body **10** through each hollow hole **331** of the supporting part **33,** preventing the urine blockage from happening.

With the foregoing configuration, the present invention achieves following advantages.

In the urinary bag reducing urine blockage provided by the present invention, the bend preventing tube **30** is disposed on one end of the catheter **20** passing through the urinary bag body **10,** and the supporting part **33** of the bend preventing tube **30** is placed inside the urinary bag body **10** to prevent the urine blockage, thereby ensuring the smooth urine drainage, improving the utility of the present invention.

The bend preventing tube **30** is integrally formed and inserted into the second connection part **22** of the catheter **20,** achieving the structural simplicity, convenient assembly, and lower manufacturing cost, so as to meet the economic efficiency.

Each hollow hole **331** is formed in a non-circular shape, which allows the urine to be more smoothly inputted into the urinary bag body **10,** so as to effectively prevent the urine blockage.

Although particular embodiments of the invention have been described in detail for purposes of illustration, various modifications and enhancements may be made without departing from the scope of the invention. Accordingly, the invention is not to be limited except as by the appended claims.

## Claims

1. An urinary bag reducing urine blockage, comprising:
an urinary bag body (10) having a through bore (12) on an upper side thereof, the through bore (12) being in communication with an interior of the urinary bag body (10);
a catheter (20) connected and in communication with the urinary bag body (10), the catheter (20) having a first connection part (21) and an opposite second connection part (22), the first connection part (21) used to be connected with an urethra of a user, the second connection part (22) passing through the through bore (12) of the urinary bag body (10) and placed inside the urinary bag body (10); and
a bend preventing tube (30) connected and in communication between the urinary bag body (10) and the catheter (20), the bend preventing tube (30) having an insertion part (32) and a supporting part (33), with a flow bore (31) passing through the insertion part (32) and the supporting part (33), the second connection part (22) of the catheter (20) mounted around the insertion part (32), the flow bore (31) in communication with the catheter (20), the insertion part (32) keeping the catheter (20) to be straight; the supporting part (33) located inside the urinary bag body (10), an outer diameter of the supporting part (33) being larger than an outer diameter of the insertion part (32), the supporting part (33) supporting the interior of the urinary bag body (10).

2. The urinary bag reducing urine blockage of claim 1, wherein the supporting part (33) is formed in an ellipsoidal shape; a plurality of hollow holes (331) are distributed on a circumferential surface of the supporting part (33); each of the hollow holes (331) is in communication with the flow bore (31).

3. The urinary bag reducing urine blockage of claim 1, wherein the through bore (12) of the urinary bag body (10) is sealed through a processing method; the processing method is applied to fix the catheter (20) to the urinary bag body (10).

4. The urinary bag reducing urine blockage of claim 3, wherein the processing method is a high-frequency welding or ultrasonic welding.

5. The urinary bag reducing urine blockage of claim 1, wherein the urinary bag body (10) comprises two fixing bores (11) passing through the upper side of the urinary bag body (10); the through bore (12) is arranged between the two fixing bores (11); two positioning bands (15) are disposed in the two fixing bores (11).

6. The urinary bag reducing urine blockage of claim 1, wherein an urine discharging tube (13) is connected to a lower middle side of the urinary bag body (10), and a valve device (14) disposed on the urine discharging tube (13).

7. The urinary bag reducing urine blockage of claim 1, wherein a plastic sheet (23) is disposed between the second connection part (22) of the catheter (20) and the urinary bag body (10).

8. The urinary bag reducing urine blockage of claim 1, wherein the bend preventing tube (30) is integrally formed of a rigid plastic material.

9. The urinary bag reducing urine blockage of claim 1, wherein the insertion part (32) comprises a first portion (321) and a second portion (322); the first portion (321) is formed in a cone shape, and the second portion (322) is formed in a column shape; a largest outer diameter of the first portion (321) is larger than an outer diameter of the second portion (322).

10. The urinary bag reducing urine blockage of claim 2, wherein each of the hollow holes (331) is formed in a shape selected from a group consisting of triangular shape, polygonal shape, or a combination thereof.
